(19) Europäisches Patentamt
European Patent Office
Office européen des brevets

(11) **EP 4 769 082 A1**

(12) **DEMANDE DE BREVET EUROPEEN**

(43) Date de publication:
01.07.2026 Bulletin 2026/27

(21) Numéro de dépôt: 25227310.7

(22) Date de dépôt: 26.12.2025

(51) Classification Internationale des Brevets (IPC):
*G06F 3/01* (2006.01)    *A61B 5/369* (2021.01)
*A61B 5/372* (2021.01)    *A61B 5/00* (2006.01)

(52) Classification Coopérative des Brevets (CPC):
**G06F 3/015; A61B 5/372; A61B 5/7267;**
A61B 5/291; A61B 2562/046

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **27.12.2024 FR 2415314**

(71) Demandeur: **Commissariat à l'Energie Atomique et aux Energies Alternatives**
**75015 Paris (FR)**

(72) Inventeurs:
• **MARTEL, Félix**
**38054 GRENOBLE CEDEX 09 (FR)**
• **AKSENOVA, Tétiana**
**38054 GRENOBLE CEDEX 09 (FR)**

(74) Mandataire: **INNOV-GROUP**
**209 Avenue Berthelot**
**69007 Lyon (FR)**

(54) **PROCÉDÉ D' APPRENTISSAGE EN LIGNE D'UNE INTERFACE NEURONALE, METTANT EN OEUVRE UN MODÈLE DE MARKOV À ÉTATS CACHÉS**

(57)    Procédé d'apprentissage d'une interface neuronale directe, l'interface neuronale directe étant reliée à des capteurs ($2_1...2_{l1}$) préalablement disposés autour du cerveau d'un utilisateur, l'interface étant configurée pour commander un actionneur (6) en fonction de signaux électrophysiologiques détectés par chaque capteur, en différentes époques temporelle, en appliquant plusieurs modèles prédictifs à un tenseur d'observation formé par les signaux détectés durant une époque temporelle. Chaque modèle prédictif est associé à un groupe d'états, chaque groupe d'état comportant des états susceptibles d'être occupés par l'utilisateur. Pour chaque groupe d'états, le modèle prédictif permet d'estimer un état de l'utilisateur en mettant en œuvre un modèle de Markov à états cachés. A chaque époque temporelle est assigné un poids. Chaque modèle prédictif est mis en œuvre ne prenant en compte le poids assigné à chaque époque, et cela dans chaque groupe d'états.

Fig. 2

**Description**

**DOMAINE TECHNIQUE**

Le domaine technique de l'invention concerne les interfaces neuronales directes, usuellement désignées par le terme « BCI » (Brain Computer Interface), destinées à commander, à partir de signaux neurophysiologiques, un actionneur.

**ART ANTERIEUR**

Le domaine des interfaces neuronales directes est en plein développement et apparaît comme une solution séduisante pour permettre à des utilisateurs en situation de handicap de commander des actionneurs par la pensée. Il s'agit de détecter et d'enregistrer des signaux électrophysiologiques émis par le cortex. Ces derniers sont traités par des algorithmes permettant de former un signal de commande, pour commander des actionneurs. Le signal de commande permet le pilotage de l'actionneur, ce dernier étant un exosquelette, un ordinateur ou un robot, pour fournir une assistance à l'utilisateur. Les algorithmes mis en œuvre permettent une traduction d'une instruction donnée par l'utilisateur, cette instruction étant captée, par des électrodes, sous la forme des signaux dits électrophysiologiques, car représentatifs de l'activité électrique des neurones. Cette activité électrique peut être mesurée au niveau du cortex, au moyen d'électrodes corticales disposées dans la boîte crânienne. Elle peut également être mesurée par des électrodes d'électroencéphalographie, moins intrusives car disposées sur le scalp, mais également moins performantes, notamment au niveau de la résolution spatiale. Une autre solution est d'enregistrer les signaux électrophysiologiques par magnétoencéphalographie, ce qui nécessite une installation dédiée.

Les algorithmes mis en œuvre sont généralement basés sur un modèle prédictif. Le modèle prédictif utilise des données d'entrée, obtenues par un prétraitement des signaux électrophysiologiques enregistrés, pour établir un signal de commande, à destination de l'actionneur ou des actionneurs. Le signal de commande doit correspondre à une intention exprimée par l'utilisateur, dont on enregistre les signaux électrophysiologiques. L'intention exprimée par l'utilisateur se manifeste sous la forme des signaux électrophysiologiques, ces derniers étant enregistrés et transmis vers l'interface neuronale directe, formant des données d'observations. Les signaux électrophysiologiques sont traités pour obtenir des données d'observations, formant des données d'entrée du modèle prédictif, ce dernier générant un signal de commande correspondant à l'intention exprimée par l'utilisateur. Le signal de commande permet une commande de l'actionneur.

Les signaux mesurés sont traitées pour former des données d'observation généralement multidimensionnelles, et comprennent :

- une composante spatiale, représentative de l'origine spatiale du signal électrophysiologique ;
- une composante fréquentielle, représentative de l'intensité du signal électrophysiologique dans différentes bandes de fréquence ;
- une composante temporelle.

Chaque donnée d'observation est associée à une époque, c'est-à-dire à un échantillon temporel de durée prédéterminée, par exemple de l'ordre de 1 seconde après que l'utilisateur ait eu l'intention d'effectuer la tâche. Le terme époque correspond à l'équivalent du terme anglosaxon « epoch ». A chaque époque, on forme un tenseur d'observation, rassemblant les données d'observation. A partir du tenseur d'observation, on alimente un modèle prédictif. Le modèle prédictif, appliqué au tenseur d'observation, permet d'estimer un signal de commande, permettant la commande des actionneurs. Le signal de commande est généralement exprimé par un vecteur de commande.

Le modèle prédictif est établi au cours d'une phase d'apprentissage, au cours de laquelle l'utilisateur effectue des tâches prédéfinies, pour lesquelles la sortie du modèle prédictif est connue. L'objectif est alors, suite à chaque tâche, de déterminer des composantes des signaux électrophysiologiques enregistrés spécifiques de la tâche. Il peut notamment s'agir de déterminer des corrélations entre des composantes des signaux électrophysiologiques et la sortie du modèle.

L'établissement de modèles prédictifs a amplement été décrit. Par exemple, le brevet US9480583 décrit l'application d'une méthode de régression linéaire des moindres carrés partiels multivariée permettant d'établir un modèle prédictif. Une telle méthode est connue sous l'acronyme anglosaxon "NPLS" ou par le terme "N-way Partial Least Squares". L'application d'une telle méthode a également été décrite dans la publication Eliseyev A, Aksenova T (2013) "Recursive N-way Partial Least Squares for Brain Computer Interface" PIOS ONE july 2013, Volume 8 Issue 7 e69962. Une telle méthode est également décrite dans le document Yelisyeyev A « Brain-Computer Interface with cortical electrical activity recording" Human health and pathology ». Université de Grenoble, 2011.

Cependant, le recours à une méthode de type NPLS nécessite de traiter un grand nombre de données d'apprentissage, par exemple plusieurs centaines ou plusieurs milliers pour une sortie du modèle correspondant à une tâche spécifique. Cela suppose un stockage en mémoire d'une quantité d'information élevée, ce qui n'est pas approprié pour effectuer un apprentissage en ligne, c'est-à-dire en temps réel, ou en quasi temps réel. Par quasi temps réel, on entend un apprentissage effectué par séquences successives, chaque séquence durant quelques secondes ou quelques minutes.

Afin de diminuer la quantité d'information à mémoriser, un procédé d'apprentissage mettant en œuvre une méthode de type REW-NPLS a été développée, REW signifiant « Recursive Exponentially Weighted », ou pondération récursive exponentielle. La formation d'un modèle prédictif, par REW-NPLS, appliqué à une interface BCI, est décrit dans EP3563218. Une telle approche est également justifiée par le fait que les signaux neuronaux ne sont pas stationnaires, ce qui impose une mise à jour régulière du modèle prédictif.

Dans la publication Moly et al « An adptative closed-loop ECoG decoder for long-term and stable bimanual control of an exoskeleton by a tetraplegic », J. Neural Eng. 19 026021, 2022, ainsi que dans EP3789852, on a décrit un algorithme de décodage de l'état d'un utilisateur, sur la base de tenseurs d'observations, en adoptant le formalisme d'une chaine d'états de Markov cachés, usuellement désignés par l'acronyme HMM (Hiden Markov Model). Le décodage est effectué selon un mélange markovien d'experts, ou MME (Markov Mixture Experts). Le décodage est effectué en utilisant un modèle de prédiction « avant », ou forward modèle, ce qui permet une mise en œuvre de la méthode en ligne. Il s'agit d'un décodage séquentiel, les états de l'utilisateur étant décodés les uns après des autres. Il peut en résulter des erreurs de décodage, ou l'exécution tâches de façon saccadée. L'avantage d'un décodage basé sur une approche markovienne est la prise en compte de probabilités de changement d'états entre deux états successifs de l'utilisateur. Ainsi, deux états successifs de l'utilisateur ne sont pas décodés de façon indépendante l'un de l'autre. L'interdépendance entre deux états successifs est considérée comme plus réaliste.

Les inventeurs proposent une alternative de la méthode décrite dans EP3789852, de façon à améliorer la performance d'apprentissage du modèle prédictif.

## EXPOSE DE L'INVENTION

Un premier objet de l'invention est un procédé d'apprentissage d'une interface neuronale directe, l'interface neuronale directe étant reliée à des capteurs préalablement disposés autour du cerveau d'un utilisateur, chaque capteur étant configuré pour détecter un signal électrophysiologique représentatif d'une activité neuronale de l'utilisateur, l'interface étant configurée pour commander un actionneur en fonction de signaux électrophysiologiques détectés, le procédé d'apprentissage comportant :

- a) sélection d'une tache mentale à exécuter par l'utilisateur, choisie parmi $I$ groupes d'états, chaque groupe d'états comportant une liste de $K_i$ tâches prédéterminées, chaque tâche d'un groupe d'états pouvant être exécutée simultanément à chaque tâche d'un autre groupe d'états ;
- b) exécution, par l'utilisateur, de la tâche sélectionnée lors de l'étape a) et au cours de l'exécution, acquisition des signaux électrophysiologiques, issus des différents capteurs, l'exécution de chaque tâche correspondant à un état de l'utilisateur, et formation, par une unité de traitement, d'un tenseur d'observation à partir de caractéristiques des signaux électrophysiologiques.
- c) réitération des étapes a) et b) durant plusieurs époques, chaque époque étant un intervalle temporel, associé à un état, les époques formant une séquence ;
- d) formation de $I$ tenseurs d'apprentissage à partir des signaux électrophysiologiques détectés à chaque époque , chaque tenseur d'apprentissage étant associé à un groupe d'états ;
- e) formation de $I$ tenseurs de commande à partir des tâches sélectionnées, chaque tenseur de commande étant associé à un groupe d'états, la valeur du tenseur de commande, à une époque, prenant une valeur inactive lorsque la tâche sélectionnée à ladite époque ne fait pas partie du groupe d'état auquel est associé le tenseur de commande ;
- f) pour chaque groupe d'états, formation d'un modèle prédictif, par régression entre le tenseur d'apprentissage et le tenseur de commande , le modèle prédictif permettant d'estimer une probabilité que l'utilisateur soit dans chaque état du groupe d'états.
- g) pour chaque groupe d'états, à partir de chaque modèle prédictif résultant de f), définition d'un modèle de Markov à états cachés, chaque modèle de Markov à états cachés étant configuré pour estimer une probabilité de l'état de l'utilisateur à chaque époque ;

les étapes c) à g) étant mises en œuvre par l'unité de traitement ;
le procédé étant caractérisé en ce qu'il comporte :

- définition d'un critère de pondération pour chaque époque ;
- dans chaque groupe d'états, assignation d'un poids à chaque époque, le poids étant défini en fonction du critère de pondération pour ladite époque, en fonction duquel à deux époques différentes, de la séquence, pour lesquelles le critère de pondération est différent, sont assignés deux poids différents ;

le procédé étant tel que dans chaque groupe d'états, la formation du modèle prédictif est effectuée en fonction du poids respectivement assigné à chaque époque.

Selon une possibilité, le poids assigné à une époque dépend de la tâche sélectionnée durant ladite époque et du groupe d'états.

Selon une possibilité :

- l'étape c) est répétée de façon à former plusieurs séquences successives, à chaque séquence étant assigné un rang chronologique;
- les étapes d) à g) sont mises en œuvre pour chaque séquence ;
- lors de l'étape f), dans chaque groupe d'états, chaque modèle prédictif est établi à partir de deux séquences consécutives, en assignant un facteur d'oubli aux données résultant de la séquence précédente.

Selon une possibilité, pour chaque groupe d'états, le critère de pondération est une fréquence d'occurrence de chaque tâche, le poids de chaque époque est d'autant plus élevé que le nombre d'occurrences de la tâche associée à l'époque, suite aux séquences successives effectuées, est faible.

Selon une possibilité, dans chaque groupe d'états, après chaque nouvelle séquence, le procédé comporte une mise à jour d'un nombre total d'occurrences pondéré pour chaque tâche, la mise à jour comportant, pour chaque tâche :

- détermination d'un nombre d'occurrences dans la nouvelle séquence ;
- pondération du nombre d'occurrences, au cours de la nouvelle séquence, par le poids respectivement assigné à la tâche dans la nouvelle séquence ;
- sommation du nombre d'occurrences pondéré de la tâche, pour la nouvelle séquence, au nombre total pondéré pour chaque tâche résultant de la séquence de rang inférieur, ce dernier étant multiplié par le facteur d'oubli.

Selon une possibilité, le critère de pondération est une performance d'apprentissage, le procédé comportant, pour chaque groupe d'états

- détermination d'un indicateur de performance d'apprentissage de chaque tâche suite à chaque époque ;
- détermination du poids de chaque tâche en fonction de l'indicateur de performance d'apprentissage de la tâche.

Selon une possibilité le critère de pondération est une qualité des signaux électrophysiologiques détectés à chaque séquence, le procédé comportant :

- détermination d'un critère de qualité des signaux collectés à chaque séquence ;
- dans chaque groupe d'états, détermination du poids de chaque tâche en fonction du critère de qualité des signaux.

Selon une possibilité, pour chaque groupe d'états, le modèle prédictif est mis en œuvre par régression multivariée, ce qui comporte un calcul d'un tenseur de covariance croisée entre le tenseur d'apprentissage et le tenseur de commande, le tenseur de covariance croisé de chaque séquence étant établi à partir d'un produit :

- du tenseur d'apprentissage;
- du tenseur de commande;
- des poids assignés à chaque époque.

Selon une possibilité :

- l'étape c) est répétée de façon à former plusieurs séquences successives, à chaque séquence étant assigné un rang chronologique (u) ;
- les étapes d) et e) sont mises en œuvre pour chaque séquence ;
- lors de l'étape f), le modèle prédictif est établi, dans chaque groupe d'états, à partir de deux séquences consécutives, à partir d'une somme du tenseur de covariance croisée établi pour la séquence de rang supérieur et du tenseur de covariance croisée établi pour la séquence de rang inférieur multiplié par un facteur d'oubli.

Selon une possibilité, dans chaque groupe d'états :

- le tenseur d'apprentissage et le tenseur de commande sont formés d'une matrice, dont une dimension est le nombre d'époques par séquence ;

- les poids forment une matrice diagonale dont chaque dimension est le nombre d'époques par séquence, chaque terme de la matrice diagonale correspondant au poids assigné à la tâche respectivement exécutée lors de ladite séquence.

Selon une possibilité, dans lequel, dans chaque groupe d'états, pour au moins une tâche spécifique, le poids est déterminé de façon que le nombre d'occurrences de ladite tâche spécifique, pondéré par le poids assigné à la tâche spécifique, soit supérieur au nombre d'occurrences d'au moins une autre tâche, pondéré par le poids assigné à ladite autre tâche.

Un deuxième objet de l'invention est une interface neuronale directe, l'interface neuronale directe comportant des capteurs préalablement disposés autour du cerveau d'un utilisateur, et configurés pour détecter des signaux électrophysiologiques, représentatifs d'une activité neuronale de l'utilisateur, l'interface étant configurée pour commander un actionneur , en mettant en œuvre un modèle prédictif, le modèle prédictif étant configuré pour générer un signal de commande de l'actionneur à partir de signaux électrophysiologiques détectés , l'interface comportant une unité de traitement, configurée pour acquérir les signaux électrophysiologiques lors de chaque étape b), et pour mettre en œuvre les étapes d) à g) d'un procédé selon le premier objet de l'invention.

L''actionneur peut être un dispositif externe à l'utilisateur ou un dispositif implantable dans le corps de l'utilisateur.

L'invention sera mieux comprise à la lecture de l'exposé des exemples de réalisation présentés, dans la suite de la description, en lien avec les figures listées ci-dessous.

## FIGURES

La figure 1 schématise une interface neuronale reliée à un utilisateur, et connectée à un processeur apte à mettre en œuvre un procédé selon l'invention.

La figure 2 représente les principales étapes d'un procédé de mise en œuvre de l'invention.

La figure 3 montre des exemples de décodages parallèles effectués en mettant en œuvre l'invention.

## EXPOSE DE MODES DE REALISATION PARTICULIER

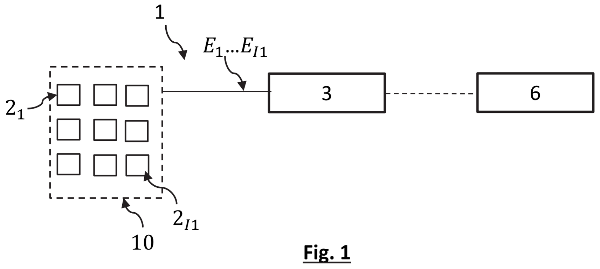

La figure 1 représente les principaux éléments d'une interface neuronale 1 selon l'invention. Il s'agit d'un dispositif comportant des capteurs $2_1...2_{I1}$, permettant l'acquisition de signaux électrophysiologiques représentatifs d'une activité neuronale. I1 est un entier correspondant au nombre de capteurs. Les capteurs $2_1...2_{I1}$ sont par exemple des électrodes corticales, l'indice I1 désignant le nombre d'électrodes corticales. Les capteurs $2_1...2_{I1}$ sont reliés à une unité de traitement 3, par exemple un microprocesseur, par une liaison filaire ou sans fil. Chaque capteur $2_1...2_{I1}$ est configuré pour détecter un signal électrophysiologique émis par un utilisateur 10. A partir de chaque signal électrophysiologique détecté, chaque capteur $2_1...2_{I1}$ transmet un signal électronique $E_1...E_{I1}$ à l'unité de traitement. L'unité de traitement 3 est apte à mettre en œuvre des algorithmes, de type modèle prédictif, pour détecter des caractéristiques des signaux électrophysiologiques $E_1...E_{I1}$ spécifiques à une tâche effectuée par l'utilisateur. L'unité de traitement 3 peut par exemple être un processeur relié à une mémoire mettant en œuvre des instructions permettant d'effectuer des algorithmes de décodage tels que décrits dans les publications citées en lien avec l'art antérieur. Ces derniers permettent de décoder les signaux physiologiques détectés de façon à déterminer les caractéristiques des signaux électrophysiologiques corrélées des tâches mentales effectuées par l'utilisateur 10.

Par tâche mentale, désignée par la suite tâche, on entend une action imaginée par un utilisateur auquel l'interface neuronale directe est raccordée. Il s'agit d'une action correspondant à une intention d'effectuer une tâche spécifique. La tâche spécifique est instruite à l'utilisateur par une tierce personne ou par un algorithme dédié.

Lors du fonctionnement opérationnel de l'interface neuronale directe 1, comme mentionné en relation avec l'art antérieur, l'utilisateur réalise successivement des tâches mentales. L'unité de traitement 3 reçoit les signaux électrophysiologiques $E_1...E_{I1}$ transmis par les capteurs $2_1...2_{I1}$, représentatifs des signaux électrophysiologiques produits par l'utilisateur et détectés par les capteurs. A partir des signaux électrophysiologiques détectés, lorsqu'une tâche est détectée, le microprocesseur génère un signal de commande $S_c$ à l'attention d'un actionneur 6. Ainsi, l'interface neuronale directe décode les signaux électrophysiologiques produits par l'utilisateur 10 de façon à générer, à l'aide d'un modèle prédictif, des signaux de commande à un actionneur. La qualité du décodage est d'autant meilleure que l'algorithme de décodage a fait l'objet d'un apprentissage de qualité.

Dans les procédés de l'art antérieur, au cours de l'apprentissage, l'utilisateur dispose d'une liste $T$ de tâches $T_k$ à effectuer. Comme décrit en lien avec l'art antérieur, au cours d'une phase d'apprentissage, un superviseur, humain ou machine, peut demander à l'utilisateur d'effectuer successivement des tâches $k$ choisies parmi la liste des $K$ tâches. L'exécution de chaque tâche correspond à un état $S_n$ dans lequel se trouve l'utilisateur à l'instant $n$. L'objectif est de déterminer progressivement les caractéristiques électrophysiologiques les mieux corrélées avec les tâches. Ces caractéristiques permettent ensuite d'établir un modèle prédictif, mis en œuvre lors du décodage, par lequel l'utilisateur 10 peut piloter l'actionneur 6 relié à l'unité de traitement 3.

Chaque tâche est à effectuer au cours d'une plage temporelle, dite époque, *n*. Le nombre d'époques à considérer pour l'apprentissage est très élevé, pouvant atteindre plusieurs centaines ou plusieurs milliers. Dans EP3563218, on a décrit une segmentation de l'apprentissage en plusieurs séquences de courte durée. Cela requiert moins de données stockées en mémoire. Le modèle de décodage, résultant d'une séquence, est ensuite mis à jour lors une séquence suivante. Cela permet d'effectuer un décodage de façon récursive, par des mises à jour successives du modèle de décodage. On prend ainsi en compte la variabilité physiologique de l'utilisateur.

Les signaux électrophysiologiques enregistrés font l'objet d'un prétraitement, selon lequel le signal de chaque électrode, durant chaque époque, fait l'objet d'une analyse fréquentielle. Il peut par exemple s'agir d'une transformée en ondelettes, par exemple une transformée en ondelette de Morlet, ou une décomposition CCWT (Continous Complex Wavelet Transform). La durée de chaque époque n peut être de 1 seconde ou 2 secondes, avec un recouvrement temporel entre deux époques consécutives. Plus précisément, durant chaque époque, une analyse fréquentielle est effectuée à intervalles réguliers, par exemple toutes les 100 ms. Une époque regroupe ainsi plusieurs analyses fréquentielles décalées dans le temps.

A chaque époque *n*, on peut associer un tenseur d'observation $\overline{X_n}$, dont :

- le premier mode correspond à la position de chaque électrode, de dimension I1.
- le deuxième mode correspond aux positions temporelles des ondelettes, de dimension I2 ;
- le troisième mode correspond aux bandes de fréquences résultant de l'analyse fréquentielle, de dimension I3 ;

Une séquence d'apprentissage *u* comporte N époques *n*, s'étendant selon une plage temporelle $\delta t$. *u* est un indice entier assigné chronologiquement à chaque séquence. A chaque séquence d'apprentissage correspond un tenseur d'apprentissage $\overline{X_u}$, de dimension NxI1xI2xI3 : le tenseur d'apprentissage $\overline{X_u}$ regroupe N tenseurs d'observation $\overline{X_n}$. D'une façon plus générale, le tenseur d'apprentissage $\overline{X_u}$ est de dimension NxI1...x Ih x...IH, avec $1 \leq h \leq H$, h étant un indice et H étant un entier positif. Dans cet exemple, H = 3.

Le terme tenseur regroupe à la fois un vecteur (tenseur d'ordre 1), une matrice (tenseur d'ordre 2) ou des tenseurs d'ordre supérieur.

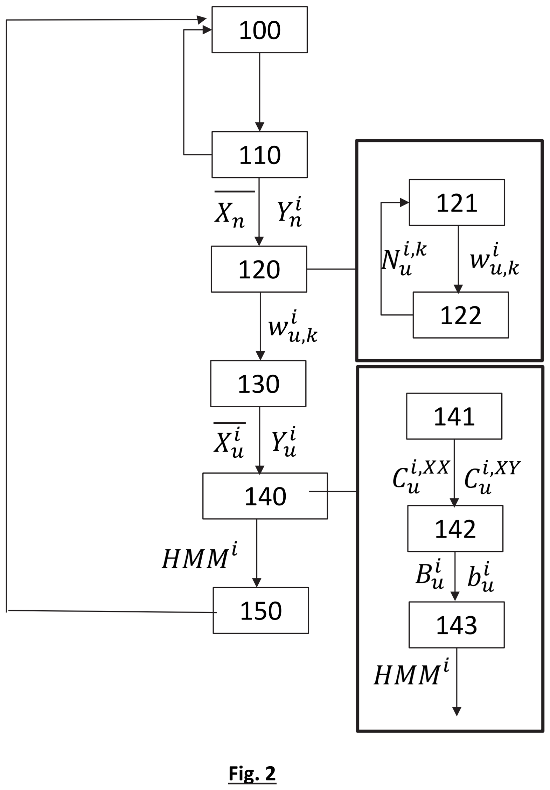

On va décrire, en lien avec la figure 2, les principales étapes de l'apprentissage d'un algorithme permettant d'estimer estimer l'état d'un utilisateur en différentes époques *n*. Sachant que le modèle prédictif est élaboré en ligne, c'est-à-dire en temps réel ou quasi-temps réel, avec une mise à jour itérative d'un modèle de régression linéaire multivarié entre les tenseurs d'observation et les instructions données à l'utilisateur. Le modèle de régression linéaire multivarié est par exemple établi par une méthode de moindres carrés partiels (NPLS), comme décrit dans EP3563218.

Les étapes impliquant un traitement mathématique sont mises en œuvre par l'unité de traitement 3.

Etape 100 : l'utilisateur imagine une tâche *k*, à un instant *t*, qui correspond à un état de l'utilisateur La tâche associée à l'instant *t* peut notamment correspondre à une action que l'utilisateur souhaite effectuer, choisi parmi plusieurs mouvements possibles. Au même instant, on enregistre les signaux électrophysiologiques résultant des différents capteurs. Plus précisément, les signaux électrophysiologiques sont enregistrés au cours d'une époque, n, s'étendant selon une durée $\delta t$ à partir de l'instant *t*.

Un aspect important de l'invention est que l'on peut demander à l'utilisateur d'effectuer simultanément plusieurs actions, par exemple plusieurs mouvements, sous réserve que les mouvements ne soient pas exclusifs les uns des autres. Deux mouvements exclusifs l'un de l'autre sont des mouvements qui ne peuvent pas être effectués simultanément. Il peut par exemple s'agir d'effectuer deux mouvements non combinables d'un même membre, par exemple ouvrir ou fermer la même main.

On dispose ainsi de *I* groupes d'états *i*, *i* étant un index entier avec $1 \leq i \leq I$. *I* peut être par exemple égal à 2. Chaque groupe d'états comporte au moins un état de repos, ainsi que d'autres états actifs de l'utilisateur qui sont exclusifs les uns des autres. Les états de groupes différents sont susceptibles d'être simultanés, et ne peuvent donc pas être exclusifs les uns des autres.

Par exemple, on définit un premier groupe d'états, relatif à la main droite, dont les états sont repos, ouverture et fermeture. On définit un deuxième groupe d'états, relatif à la main gauche, dont les états sont également repos, ouverture et fermeture.

L'invention repose sur une utilisation simultanée de plusieurs modèles de Markov cachés, différents l'un de l'autre. Ainsi, contrairement à ce qui a été décrit dans l'art antérieur, lors de l'apprentissage, à chaque époque *n*, on forme simultanément plusieurs vecteurs de commande $Y_n^i(k)$ respectivement associés à chaque groupe d'états. Dans l'exemple considéré, chaque vecteur de commande est un vecteur comportant 3 termes. De façon plus générale, chaque signal de commande peut être une matrice, ou un tenseur d'ordre supérieur. Différents signaux de commandes associés à deux groupes d'état différents peuvent avoir des dimensions différentes.

Lors de chaque époque, un état est déterminé dans chaque vecteur de commande $Y_n^i$. Chaque terme $Y_n^i(k)$ du vecteur de commande correspond à un état $k$. La valeur de $Y_n^i(k)$ est égale à 1 lorsque l'utilisateur imagine qu'il exécute le mouvement $k$ et 0 autrement.

A chaque époque n correspond un vecteur de commande $Y_n^i$ de dimension ($K_i$,1). $K_i$ est le nombre d'états possibles dans le groupe d'états $i$. Chaque terme du vecteur de commande correspond à une tâche $T_k$, parmi la liste $T^i$ des $K_i$ tâches prédéfinies dans le groupe d'états $i$. Au cours des $N$ époques formant la plage temporelle $u$, les différents signaux de commande forment une matrice $Y_u^i$ de dimension ($K_i$,N).

Etape 110 : Pré-traitements. A chaque époque n, les signaux font l'objet d'une analyse temps-fréquence, comme décrit ci-avant, de façon à former un tenseur d'observation $\overline{X_n}$.

Les étapes 100 et 110 sont répétées $N$ fois, de façon à former un tenseur d'apprentissage $\overline{X_u}$. $N$ peut par exemple être égal à 150. $N$ est le nombre d'époques $n$ formant la séquence d'apprentissage $u$. par la suite, $u$ désigne une séquence, c'est-à-dire une succession d'instants d'époques $n$. Par exemple, la durée totale des étapes 100 à 110 peut être de 15 secondes, chaque époque durant une durée $\delta t$ de 1 seconde, avec un décalage de 100 ms entre deux époques successives $n$, $n$+1, ce qui implique un recouvrement de 90% entre deux époques successives.

Etape 120 : Assignation d'un poids à chaque classe.

On a indiqué que chaque groupe d'états comporte un état de repos (Idle State - IS). Les inventeurs ont constaté que la segmentation des états en plusieurs groupes d'états peut entraîner, une surreprésentation de l'état de repos au cours de l'apprentissage. Par exemple, lorsque l'utilisateur effectue un apprentissage d'états représentant des mouvements de la main droite, le signal de commande correspondant à la main gauche est uniquement formé d'états de repos. Ainsi, au sein de chaque groupe d'états, la tâche de repos est surreprésentée, au détriment d'autres états. Pour les états sous représentés, la durée d'apprentissage plus longue.

Afin d'équilibrer l'apprentissage, à chaque époque $n$, et pour chaque groupe d'états $i$, on assigne un poids $w_n^i$, dont la valeur varie selon que l'on souhaite surpondérer ou sous-pondérer l'observation à ladite époque $n$, en fonction de l'état associé à ladite époque. Il peut s'agir, par exemple, de surpondérer des états actifs, correspondant à une activité, par rapport à un état de repos, dans un même groupe d'états. Alternativement, on peut sous-pondérer certains états par rapport à d'autres. Dans chaque groupe d'états, le poids $w_n^i$ dépend de la tâche $k$ assignée à l'époque $n$, parmi les $K_i$ tâches possibles. Dans chaque groupe d'états $i$, la tâche $k$ assignée à l'époque $n$, correspond au terme non nul du signal de commande $Y_n^i$. Au cours d'une même séquence $u$, et dans un même groupe d'états $i$, les poids $w_n^i$ correspondant à une même tâche $k$, c'est-à-dire une même tâche, ont la même valeur.

Sous-Etape 121 : Détermination des poids $w_n^i$

Suite à une séquence $u$, dans chaque groupe d'états $i$, les poids $w_n^i$ sont établis comme suit :

On détermine le nombre d'occurrences $N_u^{i,maj}$ de l'état majoritaire suite à la séquence $u$ :

$$N_u^{i,maj} = \max_k ( \lambda N_{u-1}^{i,k} + n_u^{i,k} ) \ (1),$$

où :

- $N_{u-1}^{i,k}$ est le nombre d'occurrences de chaque état $k$ suite à la séquence précédente $u$ - 1. Lors de la première séquence, $N_{u-1}^{i,k}$ est initialisé, par exemple égal à 0.
- $n_u^{i,k}$ est le nombre d'occurrences de chaque état $k$, au cours de la séquence $u$, dans le groupe d'états $i$, avant la pondération.
- $\lambda$ est un facteur d'oubli, de préférence compris entre 0 et 1.

On détermine le poids $w_u^{i,k}$ assigné à chaque état $k$, du groupe d'états i, durant la séquence $u$, par :

$$w_u^{i,k} = \frac{N_u^{i,maj} - \lambda N_{u-1}^{i,k}}{n_u^{i,k}} \quad (2)$$

et

$$w_u^{i,k} = 0 \text{ si } n_u^{i,k} = 0 \quad (3)$$

Il est préférable que des poids trop importants ne soient pas assignés, de façon à ne pas augmenter le niveau de bruit affectant la détermination du modèle prédictif. Cela revient à éviter une surpondération de certains états $k$. Ainsi, on peut imposer qu'une valeur maximale $w_{max}^i$ soit établie . Lorsque (2) conduit à une valeur $w_u^{i,k}$ telle que $w_u^{i,k} \geq w_{max}^i$ , alors $w_u^{i,k} = w_{max}^i$ .

Après que le poids $w_u^{i,k}$ assigné à chaque état $k$ a été défini, le poids $w_n^i$ associé à l'époque $n$ est tel que $w_n^i = w_u^{i,k}$ , $k$ correspondant à l'état associée à l'époque n dans le groupe d'états $i$.

La sous-étape 121 est effectuée pour chaque groupe d'états $i$.

<u>Sous-étape 122</u> : détermination de $N_u^{i,k}$ Dans chaque groupe d'états $i$, On détermine $N_u^{i,k}$ , qui correspond au nombre d'occurrences pondéré de la classe $k$, par :

$$N_u^{i,k} = \lambda N_{u-1}^{i,k} + w_{u,k}^i n_u^{i,k} \quad (4)$$

$N_u^{i,k}$ est destiné à être utilisé lors de la mise en œuvre des expressions (1) et (2) lors de la séquence suivante $u + 1$.

Outre la fréquence d'occurrences des tâches, d'autres critères peuvent être pris en compte pour assigner un poids à chaque époque .

- la performance d'apprentissage : on peut par exemple sous-pondéré les tâches pour lesquels la performance d'apprentissage est considérée comme faible. La qualité d'apprentissage peut être évaluée par un indicateur de performance de type rappel (recall), qui correspond à un ratio entre le nombre d'occurrences de tâches correctement classées sur le nombre de tâches indiquées à l'utilisateur.
- la présence d'un outlier (valeur aberrante) à l'époque considérée , auquel cas le poids peut être choisi nul : il s'agit ici d'assigner un poids en fonction de la qualité des signaux enregistrés, de façon à minimiser ou annuler l'influence de signaux considérés comme aberrants;
- la survenue d'un changement de tâche, en sous-pondérant les instants survenant juste après un changement de tâche étant sous-pondérés par rapport aux instants postérieurs : il s'agit de prendre en compte un temps de réaction de l'utilisateur, survenant à chaque changement de tâche, et au cours duquel la réponse neurologique de l'utilisateur est considérée comme transitoire;

D'une façon plus générale, un critère de pondération est défini, pour chaque époque. Il peut s'agir d'un critère de fréquence d'occurrence de la tâche effectuée à chaque époque, ou de performance d'apprentissage de la tâche sélectionnée à chaque époque, ou un critère de qualité des signaux enregistrés à chaque époque, ou un critère temporel suite à un changement de tâche. Différents critères de pondération peuvent être combinés.

<u>Etape 130</u> : normalisation des tenseurs d'apprentissage

A chaque séquence d'apprentissage $u$ correspond un tenseur d'apprentissage $\overline{X_u}$, de dimension $N$x$I1$x$I2$x$I3$ : le tenseur d'apprentissage $\overline{X_u}$ regroupe $N$ tenseurs d'observation $\overline{X_n}$ correspondant respectivement à $N$ époques $n$. Chaque tenseur d'observation $\overline{X_n}$ est formé de termes $\overline{X_n}(j), j = (j_1 \ldots \ldots jH)$ est une coordonnée multidimensionnelle dans le tenseur d'observation et H est le nombre de modes du tenseur d'observation.

Chaque tenseur d'observation $\overline{X_n}$ est normalisé, la normalisation étant spécifique à chaque groupe d'états $i$.

$$N_u^{i,Tot} = \lambda N_{u-1}^{i,Tot} + \sum_{n=1}^{N} w_n^i \quad (5)$$

- $N_u^{i,Tot}$ est un terme de normalisation pour la séquence $u$ ; $N_u^{i,Tot}$ est la taille de la base d'apprentissage accumulée depuis le début de l'apprentissage en prenant en compte les poids.
- $\lambda$ est le facteur d'oubli précédemment décrit ;
- $w_n^i$ est le poids associé à chaque époque n de la séquence $u$ ;
- $N_{u-1}^{i,Tot}$ est le terme de normalisation pour la séquence précédente $u$ - 1.Lors de la première séquence ($u$ = 1), on prend $N_0^{i,Tot} = 0$.

On calcule ensuite une moyenne $\mu_u^{i,j}$ pour chaque terme de coordonnée $j$ des $N$ tenseurs d'observation, formant la séquence $u$. La moyenne prend en compte les séquences précédentes.

$$\mu_u^{i,j} = \frac{1}{N_u^{i,Tot}} (\lambda N_{u-1}^{i,Tot} \mu_{u-1}^{i,j} + \sum_{n=1}^{N} w_n^i \overline{X_n} (j) )(6)$$

$\overline{X_n}(j)$ est chaque terme de coordonnée $j$ du tenseur d'observation $\overline{X_n}$;

On calcule ensuite une somme quadratique $SS_u^{i,j}$ :

$$SS_u^{i,j} = \lambda SS_{u-1}^{i,j} + \sum_{n=1}^{N} w_n^i \overline{X_n}(j)^2 \ (7)$$

On calcule ensuite un écart type

$$\sigma_u^{i,j} = \sqrt{\frac{SS_u^{i,j} - N_u^{i,Tot} \mu_u^{i,j^2}}{N_u^{i,Tot} - 1}} \ (8)$$

Et on normalise chaque terme $\overline{X_n}(j)$ du tenseur d'observation par :

$$\overline{X_n}(j)^i \ \leftarrow \ \frac{\overline{X_n}(j)^i - \mu_u^{i,j}}{\sigma_u^{i,j}} \ (9) \leftarrow \text{signifie « est remplacé par »}$$

On obtient ainsi autant de tenseurs d'observation $\overline{X_n}^i$ normalisés que de groupes d'état $i$, en utilisant les poids $w_n^i$ définis à chaque époque $n$, pour chaque groupe d'états. L'exposant * matérialise le fait que le tenseur est normalisé.

Pour chaque groupe d'états $i$, Les tenseurs d'observation normalisés $\overline{X_n}^i$ sont regroupés pour former un tenseur d'apprentissage normalisé $\overline{X_u}^i$ correspondant à la séquence $u$, et cela pour chaque groupe d'états $i$.

On procède de la même façon pour chaque vecteur de commande $Y_n^i$ respectivement défini pour chaque groupe d'états et pour chaque époque avec

On calcule une moyenne $\mu_u^{i,k}$ pour chaque terme des $N * I$ vecteur de commande $Y_n^i$ pour la séquence $u$, en prenant en compte les séquences précédentes.

$$\mu_u^{i,k} = \frac{1}{N_u^{i,Tot}} (\lambda N_{u-1}^{i,Tot} \mu_{u-1}^{i,k} + \sum_{n=1}^{N} w_n^i Y_n^i (k)) \ (10)$$

$Y_n^i (k)$ est un terme de coordonnée $k$ du vecteur de commande $Y_n^i$ ;

On calcule ensuite une somme quadratique $SS_u^{i,k}$ :

$$SS_u^{i,k} = (\lambda SS_{u-1}^{i,k} + \sum_{n=1}^{N} w_n^i Y_n^i (k)^2 )(11)$$

On calcule ensuite un écart type

$$\sigma_u^{i,k} = \sqrt{\frac{SS_u^{i,k} - N_u^{i,Tot} \mu_u^{i,k^2}}{N_u^{i,Tot} - 1}} \quad (12)$$

$$Y_n^i(k) \leftarrow \frac{Y_n^i(k) - \mu_u^{i,k}}{\sigma_u^{i,k}} \quad (13)$$

L'étape 130 revient à normaliser chaque tenseur d'observation $\overline{X_n}^i$ et chaque tenseur de commande $Y_n^i$ en prenant en compte le poids $w_n^i$ associé à chaque époque *n* de la séquence *u* pour le groupe d'états *i*. Il s'agit de calculer une moyenne temporelle et un écart type temporel, pondérés par le poids assigné à chaque époque, de chaque terme des tenseurs d'observation et du vecteur de commande. La moyenne temporelle et l'écart type temporel sont calculés pour les termes de mêmes coordonnées, en prenant en compte chaque époque *n* formant la séquence *u*, ainsi que les époques précédentes, via le facteur d'oubli λ.

Chaque tenseur d'observation normalisé $\overline{X_n}^i$ peut être exprimé sous la forme d'un vecteur d'observation $X_n^i$, de dimension P, avec P = I1 x I2 x I3, suite à une vectorisation du tenseur $\overline{X_n}^i$, auquel cas le tenseur d'apprentissage $\overline{X_u}^i$ est une matrice d'apprentissage $X_u^i$ formée à partir des *N* vecteurs d'observation normalisés : $X_u^i = \left(X_{n=1}^i, \dots X_{n=N}^i\right)^T$. La matrice d'apprentissage ${X_u^i}^T$ est de dimension (*N, P*).

On forme également une matrice de commande $Y_n^i$ à partir de chaque vecteur de commande.

$Y_u^i = \left(Y_{n=1}^i, \dots Y_{n=N}^i\right)^T$. Dans cet exemple, $Y_u^i$ est de dimension (*N, K_i*).

On dispose ainsi, pour chaque groupe d'états, et pour la séquence *u*, de couples $X_u^i, Y_u^i$.

<u>Etape 140</u> : paramétrage du modèle Markoviens d'états cachés.

<u>Sous-étape 141</u> : calcul des tenseurs de covariance

A partir de la matrice d'apprentissage $X_u^i$ et de la matrice de commande $Y_u^i$ résultant de l'étape 130, les matrices de covariance et de covariance croisées sont calculées selon :

$$C_u^{i,XX} = X_u^{i,T} diag(W_u^i) X_u^i + \lambda C_{u-1}^{i,XX} \quad (20)$$

et

$$C_u^{i,XY} = X_u^{i,T} diag(W_u^i) Y_u^i + \lambda C_{u-1}^{i,XY} \quad (21)$$

$diag(W_u^i)$ est une matrice diagonale de dimension (*N,N*). Chaque terme de $diag(W_u^i)$ est le poids $w_n^i$ assigné à l'époque *n*, pour le groupe d'états *i*, calculé lors de l'étape 120.

Les matrices de covariance et de covariance croisée sont utilisées pour établir la régression linéaire multivariée, comme décrit dans la sous-étape suivante.

<u>Sous-étape 142</u> : Détermination de paramètres de régression pour chaque état *i*.

Au cours de cette étape, on établit un modèle de régression linéaire multivariée *F^i*, par un algorithme récursif de type REW-NPLS, en projetant le tenseur d'apprentissage dans un espace latent de faible dimension, maximisant la covariance entre les tenseurs d'apprentissage et de commande.

Comme décrit dans la publication Moly, citée dans l'art antérieur, et plus précisément dans le paragraphe 2.3, on peut établir, à chaque époque *n*, des paramètres du modèle de régression $B_u^i$ et $b_u^i$ tels qu'à chaque instant *n* de la séquence *u* :

$$\hat{Y}_n^i = B_u^i \overline{X_n}^i + b_u^i \ (22)$$

- $B_u^i$ est une matrice de coefficients de régression de dimension ($K_i$, $P$) ;

- $b_u^i$ est un vecteur de biais de dimension ($K_i$, 1).

La détermination des paramètres du modèle de régression $B_u^i$ et $b_u^i$ est par exemple décrite dans l'étape 140 de EP3563218. $B_u^i$ et $b_u^i$ sont réactualisés de façon récursive lors de chaque séquence d'apprentissage $u$, en prenant en compte les paramètres $B_{u-1}^i$ et $b_{u-1}^i$ de la séquence précédente. Dans EP3563218, le modèle prédictif de commande est mis à jour par régression linéaire multivariée par moindres carrés partiels (NPLS), mais d'autres types de régressions multivariées peuvent être utilisables.

<u>Sous-étape 143</u> : détermination d'un modèle markovien d'états cachés *HMM$_i$* pour chaque groupe d'états *i*.

Le modèle de régression *F$^i$* défini pour chaque groupe d'états peut être utilisé pour estimer un état le plus probable de l'utilisateur durant l'époque *n*, connaissant les observations antérieures.

A partir du modèle de régression, on détermine, chaque groupe d'états *i*, un vecteur probabilité d'émission de dimension ($K_i$,1) dont chaque terme, pour chaque état k du groupe, est :

$$p\left(S_n^i = k \middle| \overline{X_n}^i\right) = softmax\left(B_u^i \overline{X_n}^i + b_u^i\right) \ (23)$$

$p\left(S_n^i = k \middle| \overline{X_n}^i\right)$ est un vecteur de dimension ($K_i$,1).

Pour un vecteur v quelconque, la fonction softmax est telle que :

$$\left(softmax(v)\right)(j) = \frac{e^{v(j)}}{\sum_l e^{v(l)}} \quad (24)$$

La fonction *softmax* peut être remplacée par une autre fonction de normalisation monotone dont l'espace d'arrivée est l'intervalle [0 ; 1].

On calcule également, dans chaque groupe d'états *i*, une matrice de transition *T$^i$*, de dimension ($K_i$, $K_i$) dont chaque terme est une probabilité de transition $p\left(S_n^i \middle| S_{n-1}^i\right)$ entre deux états successifs à des époques *n*-1 et *n*. La matrice de probabilité de transmission est déterminée en fonction du nombre de transitions entre deux états successifs durant l'apprentissage, en prenant en compte l'ensemble des séquences.

Chaque terme de la diagonale de la matrice de transition *T$^i$* est une probabilité de transition d'un état vers lui-même.

La probabilité de transition entre deux états successifs est un vecteur de dimension ($K_i$,1) tel que :

$$p_{n-1;n}^i = p\left(S_{n-1}^i, \bar{X}_{1:n-1}^i\right)T^i \ (25)$$

- $p_{n-1;n}^i$ est un vecteur de dimension ($K_i$,1) ;

- $p\left(S_{n-1}^i, \bar{X}_{1:n-1}^i\right)$ est déterminée lors de l'itération précédente, à l'instant *n*-1.

Lors de la première itération, $p\left(S_0^i, X_{1:0}\right)$ peut être choisie équiprobable et égale à 1/$K_i$

$$p\left(X_n \middle| S_n^i = k\right) = \frac{p\left(S_n^i = k \middle| X_n\right)p(X_n)}{p(S_n^i = k)} \ (26)$$

Et, selon (23),

$$p\big(S_n^i = k \big| X_n\big) = softmax\left(B_u^i \,\overline{X_n}^i + b_u^i\right) \ (27)$$

$p(X_n)$ est un coefficient multiplicatif commun à tous les états et $p(S_n^i = k)$ (probabilité à priori) est également une constante.

On pose

$$p\big(S_n^i = k, X_{1:n}\big) = p\big(X_n \big| S_n^i = k\big) \Sigma_l(p(S_{n-1}^i = l, X_{1:n-1})p(S_n^i = k \big| S_{n-1}^i = l)) \ (28)$$

Sachant que :

- $p\big(S_{n-1}^i = l, X_{1:n-1}\big)$ est déterminé lors de l'itération précédente, à l'époque *n*-1;

- $p\big(X_n \big| S_n^i = k\big)$ est déterminé par (26).

On calcule ensuite

$$p\big(S_n^i = k \big| X_{1:n}\big) = \frac{p(S_n^i = k, X_{1:n})}{\Sigma_l\, p(S_n^i = l, X_{1:n-1})} \ (29)$$

$p\big(S_n^i \big| X_{1:n}\big)$ est un vecteur de dimension ($K_i$,1), défini pour chaque groupe d'états *i*.

L'état de l'utilisateur durant l'époque n est ensuite défini en combinant l'ensemble des vecteurs $p\big(S_n^i \big| X_{1:n}\big)$. Il s'agit de l'état *k* maximisant $p\big(S_n^i \big| X_{1:n}\big)$ pour l'ensemble des groupes d'état *i*.

Etape 150 : réitération. Les étapes 100 à 140 sont réitérées pour une séquence d'apprentissage suivante *u*+1, ce qui permet une mise à jour régulière de chaque modèle HMM^i.

Variante

Selon une possibilité, il est avantageux, durant l'apprentissage, de volontairement viser un déséquilibre spécifique entre les occurrences des états de l'utilisateur. Cela peut notamment concerner un état prédéterminé, par exemple l'état de repos. Un entraînement insuffisant de l'état de repos peut générer, lors de la mise en œuvre des modèles HMM^i, des fausses activations, selon lesquelles l'utilisateur est considéré comme dans un état actif alors que l'état souhaité est un état de repos.

Il s'agit ici de privilégier l'apprentissage de l'état de repos, sachant que la pondération précédemment évoquée vise à ne pas trop fortement déséquilibrer l'apprentissage de l'état de repos par rapport aux autres états actifs.

On peut par exemple viser une proportion d'occurrences plus élevée pour l'état de repos que pour les autres états actifs du même groupe d'états. Pour cela, pour chaque état *k* de chaque groupe d'état *i*, on assigne une proportion cible $R_k^i$. Par exemple, si l'on dispose de trois états, parmi lesquels 2 états actifs et l'état de repos, les proportions cibles de chaque état actif peuvent être de 0.25, et la proportion cible de l'état de repos peut être de 0.5.

Dans la sous-étape 121, la proportion cible $R_k^i$ .est prise en compte comme suit :

$$N_u^{i,maj} = \max_k\left(\frac{\lambda N_{u-1}^{i,k} + n_u^{i,k}}{R_k^i.}\right) \ (1')$$

et

$$w_{u,k}^i = \frac{N_u^{i,maj} R_k^i - \lambda N_{u-1}^{i,k}}{n_u^{i,k}} \ (2')$$

Les inventeurs estiment qu'il est préférable que dans chaque groupe d'états, la classe de repos soit sur-représentée d'un facteur 2 à 2.5. Cela améliore la performance ainsi que la stabilité du décodage.

D'autres variantes de pondération sont décrites dans la demande FR2415310 déposée le 27/12/2024.

Essais expérimentaux

Des signaux EcoG ont été enregistrés à l'aide d'un implant sans fil WIMAGINE tel que décrit dans Mestais C. et al « WIMAGINE : Wireless 64-Channel ECoG recording implant for long term clinical applications », IEEE Transactions on neural systems and rehabilitation engineering, Vol. 23, No1 , January 2015.

Un tenseur d'observation a été calculé toutes les 100 ms, selon une fenêtre glissante. L'analyse fréquentielle a été effectuée par transformée en ondelettes complexe continue (CCWT) sur la dernière seconde de signal, avec quinze ondelettes dérivées de l'ondelette mère de Morlet, et centrées sur quinze fréquences également espacées entre 10 et 150 Hz.

Chaque séquence était formée d'une mémoire tampon (buffer) stockant les données résultant de chaque capteur durant 15 secondes. Cela correspond à un jeu de 150 données d'observation par buffer. La durée d'une époque était de 1 seconde.

On a mis en œuvre les étapes précédemment décrites en prenant en compte deux groupes d'états tels que décrits ci-dessous.

Tableau 1

| Consigne donnée à l'utilisateur | Groupe 1 (main gauche) | Groupe 2 (main droite) |
|---|---|---|
| Repos | Repos | Repos |
| Fermeture main gauche | Fermeture main gauche | Repos |
| Ouverture main gauche | Ouverture main gauche | Repos |
| Fermeture main droite | Repos | Fermeture main gauche |
| Ouverture main droite | Repos | Ouverture main droite |
| Fermeture des deux mains | Fermeture main gauche | Fermeture main gauche |
| Ouverture des deux mains | Ouverture main gauche | Ouverture main droite |

La durée de l'apprentissage était de 38 minutes, soit 152 séquences

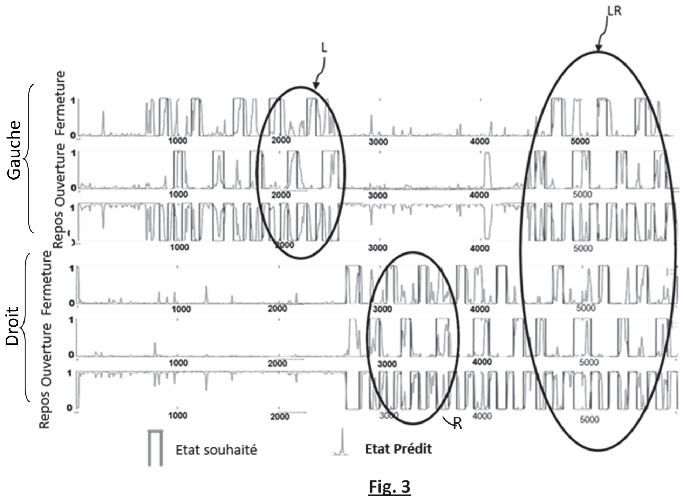

La figure 3 représente la séquence des états ordonnés à l'utilisateur (consigne), ainsi que les états décodés en mettant en œuvre la méthode précédemment décrite, pour la main gauche et pour la main droite. On observe que la méthode permet de décoder des mouvements séquentiels exclusifs de la main gauche (voir par exemple les états repérés « L ») et de la main droite (cf. états repérés R) , ainsi que des mouvements simultanés des deux mains (états repérés « LR »).

Le tableau 2 représente la matrice de confusion obtenue. Les labels en colonne correspondent à la vraie classe - les labels sur la première ligne correspondent à la classe prédite.

Tableau 2

| | Repos | Ouverture main gauche | Ouverture main droite | Fermeture main gauche | Fermeture main droite | Ouverture deux mains | Fermeture deux mains |
|---|---|---|---|---|---|---|---|
| Repos | 0.79 | 0.07 | 0.04 | 0.06 | 0.02 | 0.01 | 0.01 |
| Ouverture main gauche | 0.42 | 0.57 | 0.00 | 0.00 | 0.00 | 0.00 | 0.00 |
| Ouverture main droite | 0.15 | 0.00 | 0.73 | 0.00 | 0.12 | 0.00 | 0.00 |
| Fermeture main gauche | 0.46 | 0.14 | 0.00 | 0.40 | 0.00 | 0.00 | 0.00 |
| Fermeture main droite | 0.41 | 0.00 | 0.05 | 0.00 | 0.53 | 0.00 | 0.00 |

(suite)

|  | Repos | Ouverture main gauche | Ouverture main droite | Fermeture main gauche | Fermeture main droite | Ouverture deux mains | Fermeture deux mains |
|---|---|---|---|---|---|---|---|
| Ouverture deux mains | 0.11 | 0.24 | 0.08 | 0.00 | 0.00 | 0.57 | 0.00 |
| Fermeture deux mains | 0.27 | 0.15 | 0.00 | 0.28 | 0.12 | 0.00 | 0.18 |

Les tâches de fermeture des deux mains et de fermeture de la main gauche ne sont pas bien apprises du fait d'un manque de données d'entraînement.

A des fins de comparaison, on a entraîné un modèle comportant un seul HMM en prenant en compte 5 états : repos, ouverture main gauche, fermeture main gauche, ouverture main droite, fermeture main droite. On a utilisé les mêmes données d'apprentissage. La matrice de confusion est représentée sur le tableau 3.

Tableau 3

|  | Repos | Ouverture main gauche | Ouverture main droite | Fermeture main gauche | Fermeture main droite | Ouverture deux mains | Fermeture deux mains |
|---|---|---|---|---|---|---|---|
| Repos | 0.82 | 0.07 | 0.04 | 0.03 | 0.04 | 0.00 | 0.00 |
| Ouverture main gauche | 0.47 | 0.52 | 0.00 | 0.01 | 0.00 | 0.00 | 0.00 |
| Ouverture main droite | 0.19 | 0.00 | 0.66 | 0.00 | 0.15 | 0.00 | 0.00 |
| Fermeture main gauche | 0.54 | 0.07 | 0.00 | 0.39 | 0.00 | 0.00 | 0.00 |
| Fermeture main droite | 0.34 | 0.01 | 0.03 | 0.00 | 0.62 | 0.00 | 0.00 |
| Ouverture deux mains | 0.05 | 0.43 | 0.49 | 0.02 | 0.00 | 0.00 | 0.00 |
| Fermeture deux mains | 0.15 | 0.12 | 0.00 | 0.42 | 0.30 | 0.00 | 0.00 |

On a également entraîné un modèle comportant un seul HMM en prenant en compte 7 états : repos, ouverture main gauche, fermeture main gauche, ouverture main droite, fermeture main droite, ouverture des deux mains, fermeture des deux mains. On a utilisé les mêmes données d'apprentissage. La matrice de confusion est représentée sur le tableau 4.

Tableau 4.

|  | Repos | Ouverture main gauche | Ouverture main droite | Fermeture main gauche | Fermeture main droite | Ouverture deux mains | Fermeture deux mains |
|---|---|---|---|---|---|---|---|
| Repos | 0.83 | 0.04 | 0.02 | 0.04 | 0.04 | 0.02 | 0.00 |
| Ouverture main gauche | 0.58 | 0.41 | 0.00 | 0.01 | 0.00 | 0.00 | 0.00 |
| Ouverture main droite | 0.19 | 0.00 | 0.59 | 0.00 | 0.21 | 0.00 | 0.00 |

(suite)

|  | Repos | Ouverture main gauche | Ouverture main droite | Fermeture main gauche | Fermeture main droite | Ouverture deux mains | Fermeture deux mains |
|---|---|---|---|---|---|---|---|
| Fermeture main gauche | 0.58 | 0.07 | 0.00 | 0.35 | 0.00 | 0.00 | 0.00 |
| Fermeture main droite | 0.39 | 0.00 | 0.05 | 0.00 | 0.57 | 0.00 | 0.00 |
| Ouverture deux mains | 0.09 | 0.28 | 0.18 | 0.00 | 0.00 | 0.45 | 0.00 |
| Fermeture deux mains | 0.3 | 0.00 | 0.01 | 0.35 | 0.33 | 0.00 | 0.01 |

On a calculé une performance globale de chaque décodage à partir des matrices de confusions. La performance globale est donnée par (quelle est la formule de calcul). Elle a été estimée à :

- 54% en mettant en œuvre l'invention (cf. tableau 2) ;
- 42.8% en mettant en œuvre le modèle HMM basé sur 5 états distincts ;
- 45.9% en mettant en œuvre le modèle HMM basé sur 7 états distincts.

L'invention pourra être mise en œuvre sur en dédiant un groupe d'états à différentes articulations, par exemple articulation du poignet, du coude, de l'épaule. Cela permet d'effectuer différents mouvements des articulations simultanément. On réduit ainsi l'obtention de mouvements saccadés résultant de la mise en œuvre d'un décodage séquentiel.
L'invention peut être mise en œuvre en utilisant un mélange markovien d'experts comme décrit dans EP4088659 L'invention peut être mise en œuvre pour la commande d'un actionneur 6 externe à l'utilisateur. Elle peut également être mise en œuvre pour établir un signal de commande appliqué à des la moelle épinière ou, par exemple selon des approches de type Stimulation Electrique Epidurale (EES : Electrical Epidural Stimulation), ou encore à des muscles ou des nerfs périphériques, par stimulation électrique fonctionnelle (Functionel Electrical Stimulation).

**Revendications**

1. Procédé d'apprentissage d'une interface neuronale directe, l'interface neuronale directe étant reliée à des capteurs ($2_1...2_E$) disposés autour du cerveau d'un utilisateur, chaque capteur étant configuré pour détecter un signal électrophysiologique ($E_1...E_{I1}$) représentatif d'une activité neuronale de l'utilisateur, l'interface étant configurée pour commander un actionneur (6) en fonction de signaux électrophysiologiques détectés, le procédé d'apprentissage comportant :

   - a) sélection d'une tache mentale ($k_i$) à exécuter par l'utilisateur, choisie parmi $I$ groupes d'états, chaque groupe d'états comportant une liste de $K_i$ tâches prédéterminées, chaque tâche d'un groupe d'états pouvant être exécutée simultanément à chaque tâche d'un autre groupe d'états ;
   - b) exécution, par l'utilisateur, de la tâche sélectionnée lors de l'étape a) et au cours de l'exécution, acquisition des signaux électrophysiologiques, issus des différents capteurs, l'exécution de chaque tâche correspondant à un état de l'utilisateur, et formation, par une unité de traitement (3), d'un tenseur d'observation à partir de caractéristiques des signaux électrophysiologiques.
   - c) réitération des étapes a) et b) durant plusieurs époques ($n$), chaque époque étant un intervalle temporel, associé à un état, les époques formant une séquence ($u$) ;

   - d) formation de $I$ tenseurs d'apprentissage ($\overline{X_u^l}$) à partir des signaux électrophysiologiques détectés à chaque époque ($n$), chaque tenseur d'apprentissage étant associé à un groupe d'états ;

   - e) formation de $I$ tenseurs de commande ($\overline{Y_u^l}$) à partir des tâches sélectionnées, chaque tenseur de commande étant associé à un groupe d'états, la valeur du tenseur de commande, à une époque, prenant une valeur inactive lorsque la tâche sélectionnée à ladite époque ne fait pas partie du groupe d'état auquel est associé le tenseur de commande ;

- f) pour chaque groupe d'états, formation d'un modèle prédictif ($F^i$), par régression entre le tenseur d'apprentissage et le tenseur de commande , le modèle prédictif permettant d'estimer une probabilité que l'utilisateur soit dans chaque état du groupe d'états.

- g) pour chaque groupe d'états, à partir de chaque modèle prédictif résultant de f), définition d'un modèle de Markov à états cachés, chaque modèle de Markov à états cachés étant configuré pour estimer une probabilité de l'état de l'utilisateur à chaque époque;

les étapes c) à g) étant mises en œuvre par l'unité de traitement ;
le procédé étant **caractérisé en ce qu'**il comporte :

- définition d'un critère de pondération pour chaque époque ;

- dans chaque groupe d'états, assignation d'un poids ( $w_n^i$ ) à chaque époque, le poids étant défini en fonction du critère de pondération pour ladite époque, de la séquence, en fonction duquel à deux époques différentes, pour lesquelles le critère de pondération est différent, sont assignés deux poids différents ;

le procédé étant tel que dans chaque groupe d'états, la formation du modèle prédictif est effectuée en fonction du poids respectivement assigné à chaque époque.

2. Procédé selon la revendication 1, tel que le poids assigné à une époque ( $w_n^i$ ) dépend de la tâche sélectionnée durant ladite époque et du groupe d'états.

3. Procédé selon l'une quelconque des revendications précédentes, dans lequel

- l'étape c) est répétée de façon à former plusieurs séquences successives, à chaque séquence étant assigné un rang chronologique ($u$) ;
- les étapes d) à g) sont mises en œuvre pour chaque séquence ;
- lors de l'étape f), dans chaque groupe d'états, chaque modèle prédictif est établi à partir de deux séquences consécutives, en assignant un facteur d'oubli aux données résultant de la séquence de rang inférieur.

4. Procédé selon la revendication 3, dans lequel pour chaque groupe d'états, le critère de pondération dépend d'une fréquence d'occurrence de chaque tâche, le poids de chaque époque est d'autant plus élevé que le nombre d'occurrences de la tâche associée à l'époque, suite aux séquences successives effectuées, est faible.

5. Procédé selon la revendication 4 comportant, dans chaque groupe d'états, après chaque nouvelle séquence ($u$), une mise à jour d'un nombre total d'occurrences pondéré pour chaque tâche ( $N_u^{i,k}$ ), la mise à jour comportant, pour chaque tâche ($k$) :

- détermination d'un nombre d'occurrences ( $n_u^{i,k}$ ) dans la nouvelle séquence ($u$) ;

- pondération du nombre d'occurrences, au cours de la nouvelle séquence, par le poids ( $w_u^{i,k}$ ) respectivement assigné à la tâche dans la nouvelle séquence ;
- sommation du nombre d'occurrences pondéré de la tâche, pour la nouvelle séquence, au nombre total pondéré pour chaque tâche ( $N_{u-1}^{i,k}$ ) résultant de la séquence de rang inférieur, ce dernier étant multiplié par le facteur d'oubli ($\lambda$).

6. Procédé selon l'une quelconque des revendications précédentes, dans lequel le critère de pondération dépend d'une performance d'apprentissage, le procédé comportant, pour chaque groupe d'états :

- détermination d'un indicateur de performance d'apprentissage de chaque tâche suite à chaque époque ;
- détermination du poids de chaque tâche en fonction de l'indicateur de performance d'apprentissage de la tâche.

7. Procédé selon l'une quelconque des revendications précédentes, dans lequel le critère de pondération dépend d'une qualité des signaux électrophysiologiques détectés à chaque séquence, le procédé comportant :

- détermination d'un critère de qualité des signaux collectés à chaque séquence ;
- dans chaque groupe d'états, détermination du poids de chaque tâche en fonction du critère de qualité des signaux.

**8.** Procédé selon l'une quelconque des revendications précédentes, dans lequel, pour chaque groupe d'états, le modèle prédictif est mis en œuvre par régression multivariée, comportant un calcul d'un tenseur de covariance croisée entre le tenseur d'apprentissage et le tenseur de commande, le tenseur de covariance croisé ( $C_u^{i\,XY}$ ) de chaque séquence est établi à partir d'un produit :

- du tenseur d'apprentissage;
- du tenseur de commande;
- des poids ( $w_n^i$ ) assignés à chaque époque.

**9.** Procédé selon la revendication 8, dans lequel :

- l'étape c) est répétée de façon à former plusieurs séquences successives, à chaque séquence étant assigné un rang chronologique ($u$) ;
- les étapes d) et e) sont mises en œuvre pour chaque séquence ;
- lors de l'étape f), le modèle prédictif est établi, dans chaque groupe d'états, à partir de deux séquences consécutives, à partir d'une somme du tenseur de covariance croisée établi pour la séquence de rang supérieur ($u$) et du tenseur de covariance croisée établi pour la séquence de rang inférieur ($u$-1) multiplié par un facteur d'oubli ($\lambda$).

**10.** Procédé selon l'une quelconque des revendications 8 ou 9, dans lequel, dans chaque groupe d'états :

- le tenseur d'apprentissage et le tenseur de commande sont formés d'une matrice, dont une dimension est le nombre d'époques par séquence ;
- les poids ( $w_n^i$ ) forment une matrice diagonale ( $diag(W_u^i)$ ) dont chaque dimension est le nombre d'époques par séquence, chaque terme de la matrice diagonale correspondant au poids assigné à la tâche respectivement exécutée lors de ladite séquence.

**11.** Procédé selon l'une quelconque des revendications précédentes, dans lequel dans chaque groupe d'états, pour au moins une tâche spécifique (*IS*), le poids est déterminé de façon que le nombre d'occurrences de ladite tâche spécifique, pondéré par le poids assigné à la tâche spécifique, soit supérieur au nombre d'occurrences d'au moins une autre tâche, pondéré par le poids assigné à ladite autre tâche.

**12.** Interface neuronale directe, l'interface neuronale directe comportant des capteurs ($2_1...2_{11}$, 5) préalablement disposés autour du cerveau d'un utilisateur, et configurés pour détecter des signaux électrophysiologiques, représentatifs d'une activité neuronale de l'utilisateur, l'interface étant configurée pour commander un actionneur (6), en mettant en œuvre un modèle prédictif de commande, le modèle prédictif étant configuré pour générer un signal de commande de l'actionneur à partir de signaux électrophysiologiques détectés , l'interface comportant une unité de traitement (3), configurée pour acquérir les signaux électrophysiologiques lors de chaque étape b), et pour mettre en œuvre les étapes d) à g) d'un procédé selon l'une quelconque des revendications précédentes, de façon à apprendre le modèle prédictif de commande.

**13.** Interface selon la revendication 12, dans lequel l'actionneur est un dispositif externe à l'utilisateur ou un dispositif implantable dans le corps de l'utilisateur

$E_1 ... E_{I1}$

**Fig. 1**

**Fig. 2**

Fig. 3

RAPPORT DE RECHERCHE EUROPEENNE

**Numéro de la demande**

EP 25 22 7310

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A,D | EP 4 088 659 A1 (COMMISSARIAT ENERGIE ATOMIQUE [FR]) 16 novembre 2022 (2022-11-16) * alinéas [0017], [0026], [0047], [0048], [0056] - [0059]; figure 3A * ----- | 1-13 | INV. G06F3/01 A61B5/369 A61B5/372 A61B5/00 |
| A,D | EP 3 789 852 A1 (COMMISSARIAT ENERGIE ATOMIQUE [FR]) 10 mars 2021 (2021-03-10) * le document en entier * ----- | 1-13 | |
| A | EP 4 024 170 A1 (COMMISSARIAT ENERGIE ATOMIQUE [FR]) 6 juillet 2022 (2022-07-06) * le document en entier * ----- | 1-13 | |

**DOMAINES TECHNIQUES RECHERCHES (IPC)**

G06F
A61B

Le présent rapport a été établi pour toutes les revendications

| Lieu de la recherche | Date d'achèvement de la recherche | Examinateur |
|---|---|---|
| Munich | 20 avril 2026 | Fournier, Nicolas |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.................................................................................
& : membre de la même famille, document correspondant

EPO FORM 1503 03.82 (P04C02)

**EP 4 769 082 A1**

## ANNEXE AU RAPPORT DE RECHERCHE EUROPEENNE
## RELATIF A LA DEMANDE DE BREVET EUROPEEN NO.

EP 25 22 7310

La présente annexe indique les membres de la famille de brevets relatifs aux documents  brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-04-2026

| Document brevet cité au rapport de recherche | | Date de publication | Membre(s) de la famille de brevet(s) | | Date de publication |
|---|---|---|---|---|---|
| EP 4088659 | A1 | 16-11-2022 | EP | 4088659 A1 | 16-11-2022 |
| | | | FR | 3122566 A1 | 11-11-2022 |
| EP 3789852 | A1 | 10-03-2021 | EP | 3789852 A1 | 10-03-2021 |
| | | | FR | 3100352 A1 | 05-03-2021 |
| | | | US | 2021064942 A1 | 04-03-2021 |
| EP 4024170 | A1 | 06-07-2022 | EP | 4024170 A1 | 06-07-2022 |
| | | | FR | 3118413 A1 | 01-07-2022 |
| | | | US | 2022207424 A1 | 30-06-2022 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 9480583 B
- EP 3563218 A
- EP 3789852 A
- FR 2415310
- EP 4088659 A

**Littérature non-brevet citée dans la description**

- **ELISEYEV A** ; **AKSENOVA T**. Recursive N-way Partial Least Squares for Brain Computer Interface. *PIOS ONE*, July 2013, vol. 8 (7), e69962
- **MOLY et al.** An adptative closed-loop ECoG decoder for long-term and stable bimanual control of an exoskeleton by a tetraplegic. *J. Neural Eng.*, 2022, vol. 19, 026021
- **MESTAIS C et al.** WIMAGINE : Wireless 64-Channel ECoG recording implant for long term clinical applications. *IEEE Transactions on neural systems and rehabilitation engineering*, January 2015, vol. 23 (1)